# EUROPEAN PATENT APPLICATION

(11) **EP 4 635 966 A1**
(43) Date of publication of application: **22.10.2025**
(21) Application number: 23844168.7
(22) Date of filing: 04.12.2023
(51) Int. Cl.: C07H 15/12, A61P 35/04, A61K 31/702

(54) **SYNTHETIC VARIANTS OF THE GANGLIOSIDE NGCGM3 AND USE THEREOF IN CANCER TREATMENT**

(30) Priority: 13.12.2022 CU 20220070
(71) Applicant: Centro de Inmunologia Molecular, Playa, Habana 11300 (CU); INSTITUTO FINLAY DE VACUNAS, La Habana 11300 (CU)
(72) Inventor: FERNÁNDEZ MOLINA, Luis Enrique, Miramar, Playa, La Habana 11300 (CU); FERNÁNDEZ MARRERO, Briandy, La Habana 17100 (CU); CLAVELL PÉREZ, Marilyn, La Habana 11300 (CU); LÓPEZ LÓPEZ, Miguel Antonio, La Habana 11500 (CU); VEREZ BENCOMO, Vicente Guillermo, La Habana 11300 (CU); TOLÓN MURGUÍA, Blanca Idelmis, La Habana 17100 (CU); RODRÍGUEZ MONTERO, María del Carmen, La Habana 11400 (CU)
(74) Representative: V.O.
(86) International application number: PCT/CU2023/050005
(87) International publication number: WO 2024/125676

(57) **Abstract**

The present invention relates to the field of biotechnology in particular cancer immunotherapy. Synthetic variants of the glycolylated sialic acid containing GM3 ganglioside (NGcGM3) are described as well as compositions and methods related to these derivatives, for using them in the treatment of malignant tumors and their metastases. According to the present invention the antitumor effect of these derivatives is attributed, at least in part, to their ability to stimulate iNKT cells.

## Description

### FIELD OF TECHNOLOGY

The present invention relates to the field of biotechnology especially in cancer immunotherapy. Synthetic variants of N-glycolylated sialic acid of GM3 ganglioside (NGcGM3) are described as well as compositions and methods of these derivatives, for the treat of malignant tumors and their metastases.

### BACKGROUND

The existence of glycosphingolipids that possess antitumor activity and act as stimulators of invariant NKT (iNKT) cells has been convincingly described in the state of the art from natural or synthetic compounds derived from α-galactosylceramide (αGalCer) (U.S. Pat. No. 5,936,076).

In contrast, for other types of glycolipids, particularly gangliosides, the evidence that they are iNKT cell-activating compounds is contradictory and poorly understood and that they possess antitumor activity is, to our knowledge, nonexistent. Experimental results are available showing that ganglioside GD3 is a tumor-associated glycolipid capable of inducing a CD1d-restricted iNKT cell response (Park et al., Immunology, 2008, 123: 145-155), whereas ganglioside GM3 appears to be inhibitory to this response, because co-immunization of mice with antigen presenting cells (APCs) pulsed with this glycolipid and GD3 prevents iNKT cell activation. Conversely, results showing how GD3 is a suppressor of the innate immune response in ovarian cancer by inhibiting the activation of iNKT cells (Webb et al. Can. Res., 2012, 72: 3744-3752), indicating how these tumors avoid the antitumor action of iNKT cells through this ganglioside, providing an early stage escape mechanism.

The same contradiction is evident from Paget et al. i.e. that the gangliosides GM3 and GD3 may be endogenous activators of iNKT cells, depending on a structural change undergone in ceramide, within a dendritic cell (DC) stimulated by a Toll receptor (TLR) agonist (Paget et al., PLoS Biology, 2019, 17 (3): e3000169). They found that both synthetic variants with acetylated sialic acid of GM3 and GD3 (NAcGM3 and NAcGD3) and with d18:1-C24:1 ceramide were able to activate iNKT cells in a CD1d-dependent manner. But Paget et al did not teach any iNKT cell activating ability of synthetic variants of GM3 containing glycolylated sialic acid (NGcGM3) nor did it show evidence of antitumor activity of NAcGM3 or NAcGD3 d18:1-C24:1.

On the other hand, it has been evidenced that NGcGM3 is a tumor neoantigen fixed by evolution and therefore of interest for cancer immunotherapy, being the basis of different strategies that have been described (Labrada et al., Semin Oncol, 2018, 45: 41-51). The prior art has supported the validation of NGcGM3 as a tumor antigen based on the experimental fact that decreasing of the expression of this ganglioside in tumor cell lines reduced the growth of solid tumors and the metastatic dissemination in mice. Of particular interest for the present invention was the decrease observed in subcutaneous tumors produced by implanting P3X63 murine myeloma cells (with high expression of NGcGM3) pre-treated with a glucosyl ceramide synthase inhibitor, showing how this ganglioside is a stimulatory factor of tumor progression (de Leon et al., Cancer Immunol Immunother,2006, 55: 443-450).

Base on this evidence no direct antitumor application of NGcGM3 ganglioside has been described; while the immunotherapeutic approaches described so far identified NGcGM3 as an antigen. Within these the closest to the present invention is the GlycoVaxGM3 vaccine, a nanoparticulated product obtained by combining NGcGM3 with the outer membrane protein complex of *N. meninigitidis.* This vaccine induces specific antibodies against the ganglioside both in experimental animals and in cancer patients (Labrada et al., Semin Oncol, 2018, 45: 41-51). However, previous art shows that this vaccine has always been obtained using a natural mixture of different molecular species of NGcGM3, obtained from equine erythrocytes (Estevez et al., Vaccine, 2000,18: 190-197).

An evidence that NGcGM3 can bind CD1d has been documented previously (Gentilini et al., Cancer Immunol Immunother, 2016, 65:551-562), which is consistent with the findings of the authors of the present invention. However, this study did not show evidence that this ganglioside is able to directly activate iNKT cells, because it started from a CD3+ cell population, purified from human peripheral mononuclear cells, which was activated in culture with αGalCer and IL-2. This procedure increased the number of iNKT cells, proving that they were able to bind to a CD1d-IgG1 fusion protein containing NGcGM3. These experiments were performed with the natural mixture of NGcGM3 so it was not possible to define whether any particular molecular species is responsible for the described effect nor do they teach any antitumor impact of the iNKT NGcGM3+ population.

### BRIEF DESCRIPTION OF THE INVENTION

An object of the present invention are synthetic gangliosides of NGcGM3 represented by formula A, possessing antitumor and anti-metastatic activity. 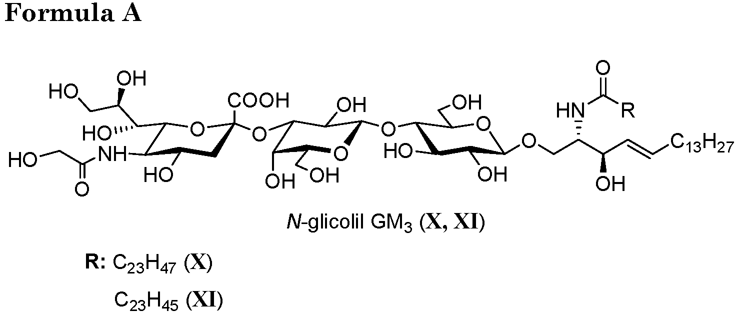 where R is selected from the group comprising:

C₂₃H₄₇

and -

C23H45

Further, the present invention relates to pharmaceutical compositions comprising as active principle the gangliosides depicted in A and a pharmaceutically acceptable vehicle. Particularly, these compositions may contain another immunomodulator. Additionally, they may have an antigen. Such compositions may be in the form of nanoparticles or liposomes. In particular, the nanoparticles are formed by hydrophobic insertion of one or more of the gangliosides described herein into hydrophobic outer membrane proteins of gram-negative bacteria, in particular *Neisseria meningitidis.* Another embodiment of the present invention is the use of the gangliosides represented in formula A or of pharmaceutical compositions containing them, in the manufacture of a medicament for the treatment of cancer and its metastases.

In a further embodiment, the present invention comprises administering to a mammal with cancer, in particular a human, a therapeutically effective amount of the gangliosides described herein or of the pharmaceutical compositions comprising them in nanoparticle or liposome form. The tumors to be treated may be CD1d positive or CD1d negative. Particularly, liver metastases are treated.

In another embodiment, the invention describes an *in vitro* method for the preparation of dendritic cells loaded with the gangliosides represented in formula A or with pharmaceutical compositions comprising them and based on: incubating dendritic cells obtained from a mammal, in particular a human, with said gangliosides and employing the obtained cells in adoptive cell transfer therapies.

### DETAILED DESCRIPTION OF THE INVENTION

The synthetic variants of interest of the ganglioside NGcGM3, according to the present invention, and as described above, are represented by formula A (i.e. formulae X and XI). The composition of the ceramides of both compounds has sphingosine in common (an aminoalcohol with 18 carbon atoms and an unsaturated hydrocarbon chain); while they differ in the fatty acid, being in compound X lignoceric acid and in the case of compound XI nervonic acid.

### Synthesis Method

For the synthesis of the NGcGM3 gangliosides represented in formula A, a procedure developed by the inventors was used. First was performing the sialylation of a hexabenzylated lactose acceptor with an N-glycolylated sialic acid donor of the thiophenyl type. After separating α anomeric trisaccharide of interest by forming its 1→4 lactone (the anomeric trisaccharide β) is not lactonized), it remains as a protective function during the rest of the sequence of synthesis steps. The synthesis process used comprises the following steps:
a) elimination of benzyl groups by hydrogenolysis
b) *per-O-acetylation* of the obtained derivative
c) selective elimination of the acetyl group attached to the anomeric carbon of the trisaccharide glucose unit
d) trichloroacetoimidate trisaccharide donor preparation
e) azido-sphingosine benzoate glycosylation
f) reduction of the azide group of the synthesized glycoside
g) acylation of the amino function formed with the carrier reagent of the second lipid chain and final elimination of the protective groups from the product obtained (including the lactone)

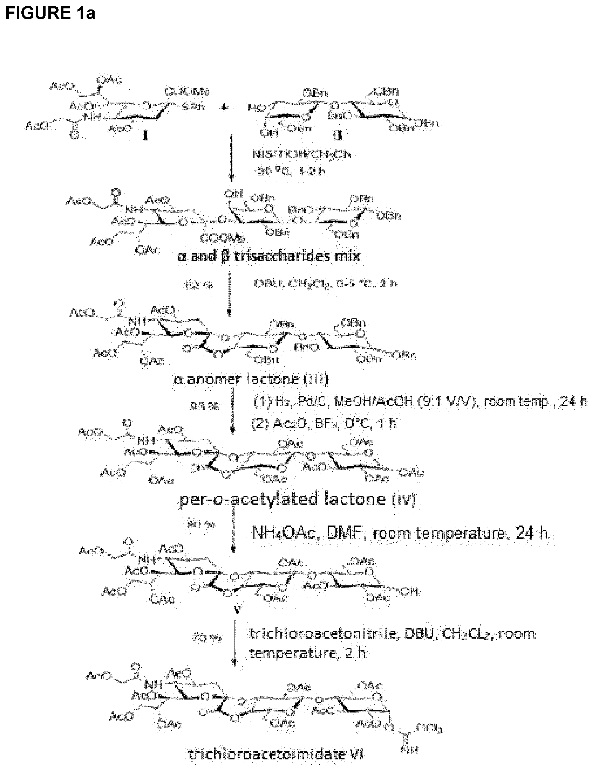

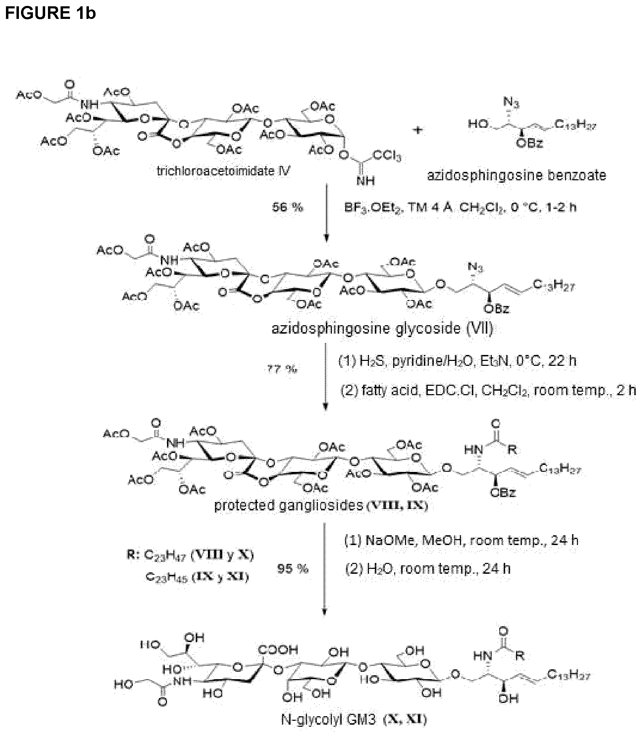

The steps of the synthesis described above for the compounds represented in formula A are better understood with the aid of Figures 1a and b. In these figures the following abbreviations were used:
**Abbreviations in chemical structures:** Ac: acetyl; Bn: benzyl; Bz: benzoyl; Me: methyl; SPh: thiophenyl.

**Abbreviations of the reagents used in the synthesis:** AcOH: acetic acid; Ac₂O: acetic anhydride; BF₃ -OEt₂ : Boron trifluoride-diethyl ether, complex; CH₃ CN: acetonitrile; DBU: 1,8-diazabicyclo [5.4.0]undec-7-ene; DMF: *N,N-dimethylformamide;* EDC: (1-ethyl-3-(3-dimethylaminopropyl) carbodiimide) hydrochloride; Et₃ N: Triethylamine; MeOH: Methanol; NaOMe: Sodium methoxide; NH₄ OAc: Ammonium acetate; NIS: *N-yodosuccinimide;* TfOH: Triflic acid.

### Origin and preparation of the lipid antigens used.

The gangliosides used were quantified by the colorimetric resorcinol assay for the detection of lipid-bound sialic acid according to the methodology described by (Svennerholm L. Biochem Biophys Acta 1957, 24: 604-11). The αGalCer (KRN7000) used as reference was purchased from either Enzo Life Science (Farmingdale, NY, USA) or Avanti Polar lipids (Alabaster, AL, USA). All lipids were initially dissolved in a 2:1 chloroform: methanol mixture and divided into aliquots of amounts suitable for use on the day. The solvent was evaporated and the dried aliquots were stored at -20°C until use. For *in vitro* experimentation, stock solutions of the glycolipids were prepared in anhydrous dimethyl sulfoxide (DMSO) plus sonication, and for *in vivo* experiments, the lipids were dissolved in a vehicle solution containing 5.6% sucrose, 0.75% L-histidine and 0.5% Tween-20.

### Hybridoma of iNKT cells

The FF13 mouse iNKT-cell hybridoma previously described by Schumann J. (Schumann J. Eur J Immunol. 2007, 37:1431-41) was provided by Dr. Lucia Mori (University Hospital Basel, Switzerland) and was cultured in RPMI-1640 medium with GlutaMAX-I and 25 mM HEPES supplemented with 10 % heat-inactivated fetal bovine serum (FBS), 100 U/ml penicillin, 100 µg/ml streptomycin and 55 µM 2-mercaptoethanol.

### Obtaining of mature dendritic cells

Mature bone marrow precursor-derived dendritic cells (bmDC) were used as APCs for *in vitro* iNKT cell activation assays and in *vivo* adoptive transfer experiments. The bmDCs were obtained according to the previously described protocol of differentiation from bone marrow progenitors in the presence of granulocyte/macrophage colony stimulating factor (GM-CSF) (Inaba K. J ExpMed.1992, 176:1693-1702) with some modifications. Briefly, bone marrow progenitors were isolated from femurs and tibias of C57BL/6 mice and cultured in complete RPMI-1640 medium (RPMI-1640 with GlutaMAX-I, 10% FBS, 100 U/mL penicillin, 100 µg/mL streptomycin and 55 µM 2-mercaptoethanol) supplemented with 20 ng/mL GM-CSF (R&D Systems). The culture was fed with fresh medium on day 3 and maturation of bmDCs was induced with 1 µg/ml LPS. On day 7, cells were harvested and used for iNKT cell activation assays or adoptive transfer.

### Obtaining mixed nanoparticles of NGcGM3 d18:1-C24:1 or NGcGM3 d18:1-C24:0 and bacterial outer membrane protein complexes (OMPCs).

Disperse OMPC of gram-negative bacteria from the group comprising *N. meningitidis, Salmonella typhi, Salmonella enteritis, Haemophilus influenzae, Bordetella pertussis or Escherichia coli,* in 0.01 M Tris-HCL buffer solution, pH 8.5, which is between 10 and 15 mM in sodium deoxycholate (DOC) and between 0.25 and 5 mM in sodium dodecylsulfate (SDS) at a final concentration between 0.5 and 3 mg/ml, in a shaking reactor for a period of 12 hours. Next, a mass of the synthetic ganglioside NGcGM3 18:1-24:1 or NGcGM3 18:1-24:0 equivalent to the added mass of OPMC is added and the agitation is prolonged for 3 to 10 hours. The detergents are then removed using a tangential filtration system with 10-100 kDa membranes. The ultrafiltrated solution is subjected to ultracentrifugation at 100 000 g for 0.5 to 2 hours. The supernatant is concentrated to adjust its concentration to the desired dosage and sterilized by filtration in a sterile capsule of 0.2 µm pore size.

The dosage used of nanoparticles to treat subjects suffering from cancer or its metastases is in the range between 10 µg and 2 mg per injection, preferably, between 30 µg and 1 mg.

### Obtaining mixed nanoparticles of NGcGM3 d18:1- C24:1 or NGcGM3 d18:1- C24:0 and bacterial outer membrane protein complexes (OMPCs) that may also contain an immunomodulator and/or a tumor antigen.

In another embodiment of the present invention, mixed nanoparticles as described above are produced, the ones which additionally contain an immune modulator selected from *Toll like receptor* (TLR) agonists 3, 7 or 9. Mixed nanoparticles are also obtained as described in the previously where tumor antigens are included, preferably neoantigens from public and private mutations.

The compounds of the present invention represented in formula A (i.e. formulae X and XI) possess antitumor, anti-metastatic and immunostimulant activity and can be used for the treatment of tumors and their metastases.

### (1) Antitumor activity

The compounds of formulae X and XI, the formulation possessing as vehicle nanoparticles obtained by inserting these gangliosides into mixtures of hydrophobic lipids or proteins from microorganisms, as well as preparations of dendritic cells pulsed with effective amounts of the gangliosides represented in formula A, exhibit antitumor activity against murine myeloma P3X63 Ag 8.653 (ATCC NCRL 1580) (X63) when cells are inoculated SQ into mice, as shown in Examples 5, 10 and 14.

### (2) Anti-metastatic activity

The compounds of formulae X and XI, the formulation possessing as vehicle nanoparticles obtained by inserting these gangliosides in mixtures of lipids or hydrophobic proteins from microorganisms, as well as preparations of dendritic cells pulsed with effective amounts of the gangliosides represented in formula A possess hepatic anti-metastatic activity against murine EL4 thymoma, when their cells are inoculated through the tail vein of mice as shown in Examples 2, 3, 9, 12, 13 and 18.

### (3) Effectiveness against CD1d-expressing human tumors

The compounds of formulae X and XI, the formulation possessing as vehicle nanoparticles obtained by inserting these gangliosides in mixtures of lipids or hydrophobic proteins from microorganisms, as well as preparations of dendritic cells pulsed with effective amounts of the gangliosides represented in formula A can be successfully used against CD1d positive hematological malignancies such as multiple myeloma, but also against CD1d expressing human solid tumors and their metastases. These include lung, head and neck, prostate, neuroblastoma and other brain tumors, melanoma, colorectal cancer and kidney carcinomas (Ingram, Z. Cells 2021, 10, 1329). Similarly, these treatments may also be effective against CD1d-negative tumors and metastases.

### BRIEF DESCRIPTION OF THE FIGURES

**Figure 1****.** Diagram of total ganglioside synthesis a) NGcGM3 d18:1-C24:1, b) NGcGM3 d18:1C24:0.
**Figure 2****.** Effect of treatment with NGcGM3 extracted from natural source and synthetic NGcGM3 d18:1-C24:1 on hepatic metastatic spread of EL-4 tumors cells.
**Figure 3****.** Effect of treatment with NGcGM3 extracted from natural source and synthetic NGcGM3 d18:1-C24:1 on survival of C57BL/6 mice with EL-4 tumors cells.
**Figure 4****.** Expression of the CD1d molecule on the cell surface of P3X63 mouse myeloma cells, measured by flow cytometry.
**Figure 5****.** Antitumor effect of NGcGM3 d18:1-C24:1 treatment over subcutaneously implanted P3X63 myeloma cells.
**Figure 6****.** Effect of different molecular species of NGcGM3 on *in vitro* activation of iNKT cells.
**Figure 7****.** Effect of different molecular species of NGcGM3 on *in vivo* activation of iNKT cells, measured by serum levels of IFNy.
**Figure 8****.** Comparison of the effect of different molecular species of NGcGM3 and GM3 with the same ceramide structure on the *in vitro* activation of iNKT cells as measured by IL-2 secretion in culture.
**Figure 9****.** Effect of treatment with nanoparticles of NGcGM3 molecular species and OMPC of *N. meningitidis* on hepatic metastatic spread of EL-4 tumors cells.
**Figure 10****.** Antitumor effect of treatment with NGcGM3 d18:1-C24:1 or nanoparticles of NGcGM3 d18:1-C24:1 and OPMC of *N. meningitidis* over subcutaneously implanted P3X63 myeloma cells.
**Figure 11****.** Effect of nanoparticles of NGcGM3 molecular species and OPMC of *N. meningitidis* on *in vitro* activation of iNKT cells measured by IL-2 secretion in culture.
**Figure 12****.** Effect of adoptive transfer of dendritic cells incubated with different molecular species of NGcGM3 on hepatic metastatic spread of EL-4 tumors cells.
**Figure 13****.** Effect of adoptive transfer of dendritic cells incubated with different molecular species of NGcGM3 on survival of C57BL/6 mice in the EL-4 tumor model.
**Figure 14****.** Effect of adoptive transfer of dendritic cells incubated with different molecular species of NGcGM3 on tumor growth of P3X63 cells.
**Figure 15****.** IFNy levels in serum of P3X63 tumor-bearing mice after the adoptive transfer of dendritic cells incubated with different molecular species of NGcGM3.
**Figure 16****.** IFNy levels in serum of EL-4 tumor-bearing mice after the adoptive transfer of dendritic cells incubated with different molecular species of NGcGM3.
**Figure 17****.** Comparison of specific antibody response against NGcGM3 induced by the injection of nanoparticles containing molecular species of NGcGM3 and OPMC of *N. meningitidis* in EL-4 tumor-bearing mice.
**Figure 18a****.** Effect of dual depletion of NK and NKT cells on the action of NGcGM3 d18:1-C24:1 in the EL-4 hepatic metastatic spread model.
**Figure 18b****.** Effect of NK cell depletion on the action of NGcGM3 d18:1C24:1 in the EL-4 cell hepatic metastatic spread model.

### EXAMPLES

The present invention will be described in detail with reference to the Experimental Examples, although it should not be assumed that it is limited to these Examples.

### Example 1. Chemical synthesis of NGcGM3 variants.

Methods for synthesizing the compounds of the present invention and their chemical-physical properties are shown below (see Figures 1a and b).

### A. Synthesis of Benzyl O-(5-acetoxyacetamido-4,7,8,9-tetra-O-acetyl-3,5-didesoxy-D-glycero-α-D-galacto-2-nonulopyranosyl-1→4-lactone)-(2→3)-O-(2,6-di-O-benzyl-βD-galactopyranosyl)-(1→4)-2,3,6-tri-O-benzyl-α/β-D-glucopyranoside (formula III) (Figure 1a).

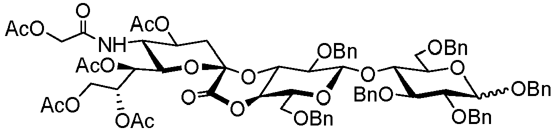

A solution of the sialic acid donor methyl(I) 5-acetoxyacetamido-4,7,8,9,10-tetra-O-acetyl-3,5-didesoxy-2-thiophenyl-D-glycero-α-D-galacto-2-nonulopyranosilonate(I) (7.25 g; 11.3 mmol) and of the lactose acceptor benzyl-O-(2,6-di-O-benzyl-β-Dgalactopyranosyl)-(1→4)-2,3,6-tri-O-benzyl-α,β-D-glucopyranoside (II)(5.8 g; 6.6 mmol) in dry CH3 CN (56 mL) was prepared, 3 Å powdered molecular sieves (8.7 g) were added and stirred for 10 min. at room temperature. The mixture was then cooled to- 30 °C, NIS (3.48 g; 15.5 mmol) was added, followed by TfOH (67 µL; 0.76 mmol) and stirred for 2 hours at the same temperature. The reaction was monitored by CCD and when finished, CH₂Cl₂ (120 mL) was added and filtered through a layer of Celite 545. The filtrate was washed with a saturated aqueous solution of Na₂S₂O until the reddish coloration disappeared and after separating the organic phase, it was neutralized with Et₃N, dried over anhydrous Na₂SO₄ and the solvents were evaporated under reduced pressure. The residue obtained contains unreacted acceptor II, the α- and β-isomers of the trisaccharide formed, as well as the elimination product of donor I.

The mixture of products obtained in the above reaction was dissolved in dry CH₂ Cl₂ (180 mL), the solution was cooled to 0 °C, DBU (2.16 mL; 14.47 mmol) was added and stirred for 2 hours at the same temperature. After neutralizing with glacial AcOH diluted in CH₂ Cl₂ (1:4 v/v), the solvent was evaporated to dryness under reduced pressure and the residue was purified by column chromatography (toluene/acetone 15:1) to recover the remaining unreacted acceptor II, followed by α [1-4]-pure lactone (III). After evaporation of the solvents, compound III was obtained as an amorphous white solid which was homogeneous by thin layer chromatography (CCD) (**R_{f}**: 0.56; toluene/acetone 2:1). Yield: 5.6 g (62 %).

**1H** NMR **(600** MHz, **CDCl₃** ): _{δ} 2.15; 2.14; 2.03; 2.02; 1.93 [(3H, s) x 5]; 7.45-7.13 (30H, m, Ar); 4.94 (2H, d, J = 11.8 Hz); 4.75 (1H, d, J = 10.7 Hz); 4.73 (1H, d, J = 10.9 Hz,); 4.65 (2H, d, J = 12.7 Hz,); 4.59 (1H, d, J = 12.1 Hz,); 4.44 (1H, d, J = 12.1 Hz); 30 4.43 (1H, d, J = 12.1 Hz); 4.31 (1H, d, J = 12.3 Hz); 4.90 (1H, d, J = 10.9 Hz); 4.89 (1H, d, J = 10.7 Hz); [12H, benzyl]; **Glc**: 4.49 (1H, d, J = 7.7 Hz, H-1); 3.46 (1H, dd, J = 9.2; 7.7 Hz, H-2); 3.56 (1H, m, H-3); 3.99 (1H, t, J = 9.4 Hz, H-4); 3.38 (1H, ddd, J = 9.8; 4.1; 1.8 Hz, H-5); 3.80 (1H, dd, J = 11.0; 4.0 Hz, H-6a); 3.73 (1H, m, H-6b); Gal: 4.47 (1H, d, J = 7.8 Hz, H-1); 3.26 (1H, dd, J = 9.3; 7.6 Hz, H-2); 4.09 (1H, dd, J = 9.4; 4.1 Hz, H-3); 4.90 (1H, m, H-4); 3.56 (1H, m, H-5); 3.73 (1H, m, H-6a); 3.44 (1H, m, H6b); **Neu:** 2.13 (1H, dd, J = 13.8; 5.4 Hz, H-3ec); 1.82 (1H, dd, J = 13.5; 11.5 Hz, H3ax); 5.48 (1H, m, H-4); 4.22 (1H, q, J = 10.4 Hz, H-5); 3.76 (1H, dd, J = 10.5; 2.1 Hz, H-6); 5.21 (1H, dd, J = 5.7; 2.1 Hz, H-7); 5.07 (1H, ddd, J = 7.2; 5.8; 3.0 Hz, H-8); 4.48 (1H, m, H-9a); 3.90 (1H, m, H-9b); 6.54 (1H, d, J = 10.3 Hz, 5-NH); 4.57 (1H, d, J = 15.2 Hz, H-10a); 4.33 (1H, d, J = 15.3 Hz, H-10b).

**MALDI-TOF MS:** [M + Na]⁺ m/z 1404.37 (calculated 1404.52).

### B. Synthesis of Acetyl O-(5-acetoxyacetamido-4,7,8,9-tetra-O-acetyl-3,5-didesoxy-Dglycero-α-D-galacto-2-nonulopyranosyl-1→4-lactone)-(2→3)-O-(2,6-di-O-acetyl-β-Dgalactopyranosyl)-(1→4)-2,3,6-tri-O-acetyl-α/β-D-glucopyranoside (formula IV) (Figure 1a).

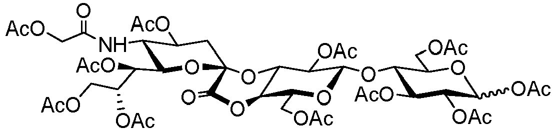

The α [1-4]-lactone **(III)** (5.6 g; 4.05 mmol) was dissolved in 230 mL of a MeOH/AcOH mixture (9:1 v/v), 10 % Pd/C (0.95 g) was added, air was purged from the balloon using an Ar (g) stream then H₂ (g) was injected and stirred for 24 hours at room temperature. After this time, the catalyst was removed by filtration through a layer of Celite 545 and the solvents were evaporated to dryness under reduced pressure. The solid obtained was suspended in Ac₂ O (5 mL), the mixture was stirred at 0 °C for 10 min, BF₃ OEt₂ (0.8 mL; 6.3 mmol) previously distilled was added and stirred for 1 hour at the same temperature. After completion of the reaction, the resulting solution was neutralized with a saturated aqueous solution of NaHCO₃, CH₂ Cl₂ was added and the phases were separated. After separation of the organic phase, the aqueous phase was washed 2 times with CH₂ Cl₂ , the organic extracts were unified, dried over anhydrous Na₂ SO₄ and the solvent was removed by evaporation under reduced pressure to give compound **IV** as a white amorphous solid which was homogeneous by CCD (**R_{f}** : 0.73; toluene/acetone 1:1). Yield: 4.1 g (93 %). A mixture of α/β anomers (7.6:3.4) was obtained. The NMR data refers to the α-anomer which was the major anomer.

**1H NMR (600 MHz, CDCl3** ):δ 2.18; 2.16; 2.12; 2.11 (2CH3 ); 2.10; 2.09; 2.05; 2.00; 1.99; 1.98 [(3H, s, CH3) x 11]; **Gluc**: 6.24 (1H, d, J = 3.7 Hz, H-1); 5.00 (1H, dd, J = 10.3; 3.8 Hz, H-2); 5.42 (1H, dd, J = 10.3; 9.4 Hz, H-3); 3.76 (1H, dd, J = 9.8 Hz, H-4); 3.99 (1H, ddd, J = 10.2; 4.6; 2.1 Hz, H-5); 4.39 (1H, m, H-6a); 4.18 (1H, dd, J = 12.2; 4.6 Hz, H-6b); **Gal:** 4.38 (1H, d, J = 8.2 Hz, H-1); 4.85 (1H, dd, J = 9.9; 8.0 Hz, H-2); 4.12 (1H, m, H-3); 4.95 (1H, dd, J = 3.8; 1.1 Hz, H-4); 3.91 (1H, m, H-5); 4.64 (1H, dd, 5 J = 12.1; 3.7 Hz, H-6a); 4.26 (1H, m, H-6b); **Neu:** 2.45 (1H, dd, J = 13.8; 5.4 Hz, H3ec); 1.79 (1H, dd, J = 13.8; 11.5 Hz, H-3ax); 5.54 (1H, td, J = 10.8; 5.3 Hz, H-4); 4.12 (1H, m, H-5); 3.68 (1H, dd, J = 10.5; 1.9 Hz, H-6); 5.15 (1H, dd, J = 9.2; 1.8 Hz, H-7); 5.19 (1H, m, H-8); 4.26 (1H, m, H-9a); 3.91 (1H, m, H-9b); 6.05 (1H, dd, J = 10.2; 1.8 Hz, 5-NH); 4.59 (1H, d, J = 15.4 Hz, H-10a); 4.26 (1H, m, H-10b).

**MALDI-TOF MS:** [M + Na]+ m/z 1116.28 (calculated 1116.30).

### C. Synthesis of 5-acetoxyacetamido-4,7,8,9-tetra-O-acetyl-3,5'-didesoxy-D-glycero-αD-galacto-2-nonulopyranosyl-1→4-lactone-(2→3)-O-(2,6-di-O-acetyl-β-Dgalactopyranosyl)-(1→4)-2,3,6-tri-O-acetyl-α/β-D-glucopyranose (formula V) (Figure 1a).

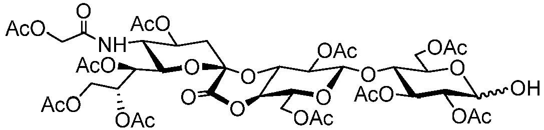

A solution of **IV** (4.1 g; 3.74 mmol) in dry DMF (20 mL) was prepared, NH₄ OAc (0.47 g; 6.19 mmol) was added and the mixture was stirred for 24 hours at room temperature. After completion of the reaction, AcOEt (100 mL) was added and washed with a saturated aqueous NaCl solution (5 x 18 mL). The organic phase was separated, dried over anhydrous Na₂ SO₄ and the solvent was removed under reduced pressure to dryness. Compound **V** was obtained as an amorphous white solid which was homogeneous by CCD (**R_{f}**: 0.59; toluene/acetone 1:1). Yield: 3.55 g (90 %). A mixture of α/β anomers (7:3) was obtained. The NMR data refers to the α-anomer which was the major anomer.

**1H NMR (600 MHz, CDCl₃** ):δ 2.19; 2.12 (3CH₃ ); 2.10; 2.09; 2.06; 2.04; 2.00; 1.99 [(3H, s, CH₃ ) x 10]; **Glc**: 5.35 (1H, d, J= 3.6 Hz, H-1); 4.83 (1H, m, H-2); 5.48 (1H, dd, J= 9.7 Hz, H-3); 3.71 (1H, m, H-4); 4.18 (1H, m, H-5); 4.43 (1H, m, H-6a); 4.18 (1H, m, H-6b); **Gal:** 4.42 (1H, d, J = 7.9 Hz, H-1); 4.83 (1H, m, H-2); 4.12 (1H, m, H-3); 4.94 (1H, dd, J= 3.7; 1.4 Hz, H-4); 3.92 (1H, m, H-5); 4.63 (1H, dt, J= 12.0; 3.4 Hz, H-6a); 4.27 (1H, m, H-6b); **Neu:** 2.46 (1H, dd, J = 13.8; 5.2 Hz, H-3ec); 1.78 (1H, dd, J= 13.9; 11.5 Hz, H-3ax); 5.53 (1H, td, J= 11.1; 5.4 Hz, H-4); 4.12 (1H, m, H-5); 3.71 (1H, m, H-6); 5.17 (2H, m, H-7 and H-8); 4.27 (1H, m, H-9a); 3.92 (1H, m, H-9b); 6.06 (1H, d, J= 10.2 Hz, 5-NH); 4.59 (1H, d, J= 15.4 Hz, H-10a); 4.27 (1H, m, H-10b).

**MALDI-TOF MS:** [M + Na]⁺ m/z 1074.11 (calculated 1074.29).

### D. Synthesis of O-(5-acetoxyacetamido-4,7,8,9-tetra-O-acetyl-3,5-didesoxy-D-glycero-α-D-galacto-2-nonulopyranosyl-1→4-lactone)-(2→3)-O-(2,6-di-O-acetyl-β-D-galactopyranosyl)-(1→4)-2,3,6-tri-O-acetyl-α-D-glucopyranosyl (formula VI) (Figure 1a).

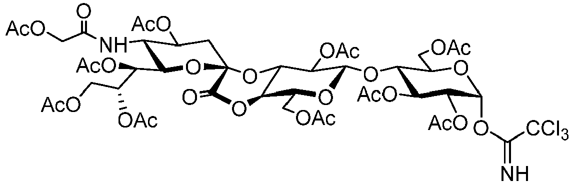

A solution of V (3.55 g; 3.37 mmol) was prepared in dry CH₂ Cl₂ (40 mL), trichloroacetonitrile (10.9 mL; 109 mmol) was added, DBU (0.52 mL; 3.46 mmol) was added dropwise and stirred for 2 hours at room temperature. Upon completion of the reaction, the mixture was evaporated to dryness under reduced pressure and the resulting residue was purified by column chromatography (CH2 Cl2 /acetone 6:1) to afford compound VI as an amorphous white solid which was homogeneous by CCD (Rf 0.61; toluene/acetone 1:1). Yield: 2.95 g (73 %).

**1H NMR (600 MHz, CDCl₃** ):δ 2.19; 2.12; 2.11; 2.10; 2.09; 2.08; 2.05; 2.00; 1.99; 1.98 [(3H, s, CH₃ ) x 10]; **Glc**: 6.47 (1H, d, J= 3.8 Hz, H-1); 5.06 (1H, dd, J= 10.1; 3.8 Hz, H-2); 5.53 (1H, m, H-3); 3.82 (1H, dd, J= 9.8 Hz, H-4); 4.11 (1H, m, H-5); 4.44 (1H, dd, J= 12.2; 2.0 Hz, H-6a); 4.19 (1H, dd, J= 12.2; 4.8 Hz, H-6b); 8.65 (1H, s, C=N); **Gal:** 20 4.41 (1H, d, J = 7.9 Hz, H-1); 4.86 (1H, dd, J= 9.9; 8.0 Hz, H-2); 4.11 (1H, m, H-3); 4.95 (1H, dd, J = 3.8; 1.2 Hz, H-4); 3.91 (1H, m, H-5); 4.65 (1H, dd, J= 12.1; 3.5 Hz, H-6a); 4.26 (1H, m, H-6b); **Neu:** 2.47 (1H, dd, J= 13.9; 5.4 Hz, H-3ec); 1.79 (1H, dd, J= 14.0; 11.5 Hz, H-3ax); 5.53 (1H, m, H-4); 4.14 (1H, d, J = 10.4 Hz, H-5); 3.69 (1H, dd, J= 10.4; 1.8 Hz, H-6); 5.15 (1H, dd, J= 9.2; 1.8 Hz, H-7); 5.19 (1H, ddd, J= 8.9; 6.0; 2.7 25 Hz, H-8); 4.26 (1H, m, H-9a); 3.91 (1H, m, H-9b); 6.02 (1H, d, J= 10.1 Hz, 5-NH); 4.60 (1H, d, J= 15.4 Hz, H-10a); 4.26 (1H, m, H-10b). **MALDI-TOF MS:** this product decomposes during the analysis and only the peak corresponding to starting compound V ([M + Na]⁺ m/z 1074.19) was observed in the spectrum.

### E. Synthesis of O-(5-acetoxyacetamido-4,7,8,9-tetra-O-acetyl-3,5-didesoxy-D-glyceroα-D-galacto-2-nonulopyranosyl-1→4-lactone)-(2→3)-O-(2,6-di-O-acetyl-β-D-galactopyranosyl)-(1→4)-2,3,6-tri-O-acetyl-β-D-glucopyranosyl-(1→1)-(2S,3R,4E)-2azido-3-benzoyl-4-octadecene-1,3-diol (formula VII) (Figure 1b).

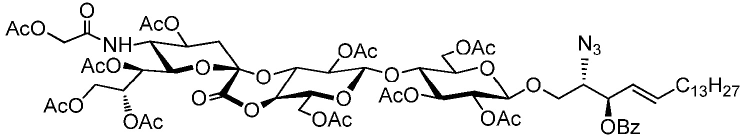

A solution of VI (2.95 g; 2.47 mmol) and azido-sphingosine benzoate (2.11 g; 4.94 mmol) was prepared in dry CH₂ Cl₂ (25 mL), powdered 4 Å molecular sieves (5 g) were added and stirred at room temperature for 30 min. The mixture was cooled to 0 °C, freshly distilled BF₃ -OEt₂ (0.65 mL; 5.17 mmol) was added and stirred for 2 hours at the same temperature. After completion of the reaction, the mixture was filtered through a layer of Celite 545, the filtrate was neutralized with Et₃N and evaporated to dryness under reduced pressure. The residue was purified by column chromatography (CH₂ Cl₂ /acetone 15:1) to recover the remaining unreacted azido-sphingosine benzoate, followed by CH₂ Cl₂ /acetone 10:1 to give pure glycoside VII as an amorphous white solid which was homogeneous by CCD (toluene/acetone 2:1, **R_{f}**: 0.58). Yield: 2.02 g (56 %).

**1H NMR (600 MHz, CDCl3 ):** δ 2,19; 2,13; 2,12; 2,10; 2,08; 2,06; 2,03 (2CH3); 2,01; 1,98 [(3H, s, CH3) x 10]; 8,05-8,00 (2H, m, orto Ar); 7,56 (1H, t, J = 7,4 Hz, para Ar); 7,44 (2H, t, J = 7,8 Hz, meta Ar); **Glc**: 4,49 (1H, d, J = 7,7 Hz, H-1); 4,94 (1H, m, H-2); 5,15 (1H, m, H-3); 3,77 (1H, dd, J = 9,5 Hz, H-4); 3,50 (1H, m, H-5); 4,44 (1H, dd, J = 11,9; 2,1 Hz, H-6a); 4,10 (1H, m, H-6b); **Gal:** 4,37 (1H, d, J = 8,0 Hz, H-1); 4,84 (1H, dd, J = 9,9; 7,9 Hz, H-2); 4,10 (1H, m, H-3); 4,94 (1H, m, H-4); 3,91 (1H, m, H-5); 4,64 (1H, dd, J = 12,0; 3,5 Hz, H-6a); 4,26 (1H, m, H-6b); **Neu:** 2,45 (1H, dd, J = 13,9; 5,4 Hz, H-3ec); 1,79 (1H, dd, J = 13,9; 11,6 Hz, H-3ax); 5,53 (1H, m, H-4); 4,14 (1H, q, J = 10,4 Hz, H-5); 3,68 (1H, dd, J = 10,5; 1,8 Hz, H-6); 5,15 (1H, m, H-7); 5,19 (1H, ddd, J = 9,0; 6,1; 2,7 Hz, H-8); 4,26 (1H, m, H-9a); 3,91 (3H, m, H-9b); 5,97 (d, J = 10,2 Hz, 5-NH); 4,60 (d, J = 15,3 Hz, H-10a); 4,26 (1H, m, H-10a); **Sph:** 3,85 (1H, dd, J = 10,5; 6,6 Hz, H-1a); 3,57 (1H, dd, J = 10,6; 6,1 Hz, H-1b); 3,91 (1H, m, H-2); 5,59 (1H, dd, J = 8,1; 4,2 Hz, H-3); 5,53 (1H, m, H-4); 5,91 (1H, dt, J = 15,3; 6,7 Hz, H-5); 2,06 (2H, m, H-6);1,40 - 1,18 (22H, m, H-7 hasta H-17); 0,86 (3H, t, J = 7,0 Hz, H-18).

**MALDI-TOF MS:** [M + Na]⁺ m/z 1485.93 (calculated. 1485.58).

### F. Synthesis of O-[5-[5-acetoxyacetamido-4,7,8,9-tetra-O-acetyl-3,5-didesoxy-Dglycero-α-D-galacto-2-nonupyranosyl-1→4-lactone)-(2→3)-O-(2,6-di-O-acetyl-β-Dgalactopyranosyl)-(1→4)-2,3,6-tri-O-acetyl-β-D-glucopyranosyl-(1→1)-(2R,3S,4E)-3benzoyl-2- tetracosanamido-4-octadecene-1,3-diol (formula VIII) (Figure 1b).

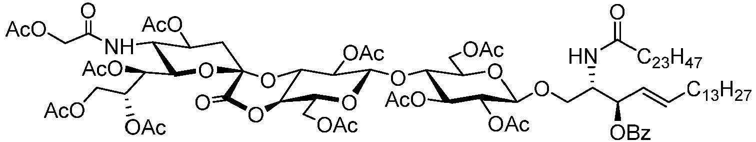

A solution of glycoside **VII** (2.02 g; 1.38 mmol) was prepared in 70 mL of a mixture of pyridine/H O/Et₂₃ N (10: 1:0.3 v/v/v/v), cooled to 0 °C, and a stream of H₂S (g) was passed through for 1 hour. The reactor was hermetically sealed, the mixture was stirred at the same temperature, and after 6 hours H₂S (g) was bubbled through the mixture for an additional hour. After sealing the reactor, the mixture was stirred for 16 hours at 0 °C. The progress of the reaction was checked by CCD and upon completion, the solvents were evaporated under reduced pressure and the residue was co-evaporated several times with toluene to remove the H₂O and pyridine residues it contains. The solid obtained was dissolved in CH₂ Cl₂ (100 mL), tetracosanoic acid (1.0 g; 2.75 mmol) was added followed by EDC-HCI (0.79 g; 4.13 mmol) and stirred for 2 hours at room temperature. After completion of the reaction, the mixture was washed with water (5 x 20 mL), the organic phase was dried over anhydrous Na₂SO₄, the solvent was evaporated under reduced pressure and the residue was purified by column chromatography using successive mixtures with different ratios of CH₂ Cl₂ /acetone (15:1→10:1) to give **VIII** as an amorphous white solid which was homogeneous by CCD (toluene/acetone 2:1, **R_{f}**: 0.58). Yield: 1.9 g (77 %).

**1H NMR (600 MHz, CDCl )**₃δ 2.19; 2.14; 2.12; 2.10; 2.06; 2.03; 2.01 (2CH₃ ); 1.98; 1.93 [(3H, s) x 10];8.03-7.97 (2H, m, Ar); 7.55 (1H, t, J = 7.4 Hz, Ar); 7.43 (1H, t, J = 7.8 Hz, Ar); **Glc**: 4.42 (1H, d, J = 7.7 Hz, H-1); 4.90 (1H, dd, J = 9.6; 7.7 Hz, H-2); 5.17-5.10 (1H, m, H-3); 3.72 (1H, dd, J = 9.5 Hz, H-4); 3.54 (1H, ddd, J = 9.9; 5.4; 2.1 Hz, H-5); 4.31-4.28 (1H, m, H-6a); 4.02-3.97 (1H, m, H-6b); **Gal:** 4.34 (1H, d, J = 7.9 Hz, H-1); 4.82 (1H, dd, J = 9.9; 7.9 Hz, H-2); 4.09 (1H, dd, J = 9.9; 3.8 Hz, H-3); 4.95-4.93 (1H, m, H-4); 3.89-3.86 (1H, m, H-5); 4.64 (1H, dd, J = 12.1; 3.4 Hz, H-6a); 4.28-4.22 (1H, m, H-6b); Neu: 2.45 (1H, dd, J = 13.9; 5.4 Hz, H-3ec); 1.78 (1H, dd, J = 13.9; 11.6 Hz, 30 H-3ax); 5.57- 5.51 (1H, m, H-4); 4.14 (1H, q, J = 10.4 Hz, H-5); 3.68 (1H, dd, J = 10.5; 1.8 Hz, H-6); 5.17-5.10 (1H, m, H-7); 5.19 (1H, ddd, J = 9.1; 6.2; 2.8 Hz, H-8); 4.28-4.22 (1H, m, H-9a); 3.92 (1H, dd, J = 12.5; 6.3 Hz, H-9b); 5.98 (1H, d, J = 10.1 Hz, 5-NH); 4.60 (1H, d, J = 15.4 Hz, H-10a); 4.28-4.22 (1H, m, H-10b); **Sph:** 3.61 (1H, dd, J = 10.1; 4.5 Hz, H-1a); 4.02-3.97 (1H, m, H-1b); 4.50-4.45 (1H, m, H-2); 5.73 (1H, d, J = 9.2 Hz, 2-NH); 5.57-5.51 (1H, m, H-3); 5.45 (1H, ddt, J = 15.3; 7.6; 1.5 Hz, H-4); 5.86 (1H, dt, J = 15.3; 6.8 Hz, H-5); 2.00 (2H, m, H-6); 1.38-1.15 (22H m, H-7 through H-17); 5 0.87 (3H, t, J = 7.0 Hz, H-18); **FA:** 2.18-2.15 (2H, m, H-2); 1.58 (2H, hept, J = 6.6 Hz, H-3); 1.38- 1.15 (40H m, H-4 through H-23); 0.87 (3H, t, J = 7.0 Hz, H-24).

**MALDI-TOF MS:** [M + Na]⁺ m/z 1809.76 (calculated 1809.94).

### G. Synthesis of O-[5-[5-acetoxyacetamido-4,7,8,9-tetra-O-acetyl-3,5-didesoxy-D-glycero-α-D-galacto-2-nonupyranosyl-1→4-lactone)-(2→3)-O-(2,6-di-O-acetyl-β-D-galactopyranosyl)-(1→4)-2,3,6-tri-O-acetyl-β-D-glucopyranosyl-(1→1)-(2R,3S,4E)-3benzoyl-2-tetracos-15-enamido-4-octadecene-1,3-diol (formula IX) (Figure 1b).

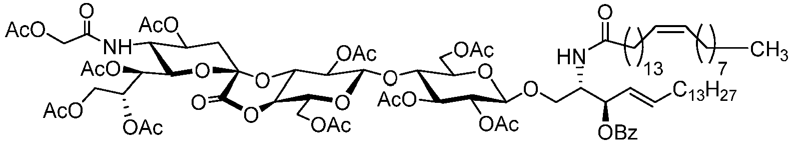

Compound IX was obtained by the procedure described above for VIII using (Z)tetracos-15-enoic acid for acylation. Yield: 1.85 g (75 %).

**1H NMR (600 MHz, CDCl )**₃δ 2.19; 2.14; 2.12; 2.10; 2.06; 2.03; 2.01 (2 CH₃ ); 1.98; 1.93 [ (3H, s) x 10];8.03-7.97 (2H, m, Ar); 7.55 (1H, dd, J= 7.4 Hz, Ar); 7.43 (2H, t, J= 7.7 Hz, Ar); **Glc**: 4.42 (1H, d, J= 7.8 Hz, H-1); 4.90 (1H, dd, J = 9.7; 7.7 Hz, H-2); 5.17-5.10 (1H, m, H-3); 3.72 (1H, dd, J= 9.5 Hz, H-4); 3.54 (1H, ddd, J= 9.9; 5.4; 2.1 Hz, H-5); 4.31-4.28 (1H, m, H-6a); 4.02-3.98 (1H, m, H-6b); Gal: 4.33 (1H, d, J= 8.0 20 Hz, H-1); 4.82 (1H, dd, J= 9.9; 7.9 Hz, H-2); 4.09 (1H, dd, J= 9.9; 3.8 Hz, H-3); 4.94 (1H, d, J= 3.9 Hz, H-4); 3.88 (1H, dd, J= 8.6; 3.4 Hz, H-5); 4.64 (1H, dd, J= 12.1; 3.4 Hz, H-6a); 4.28-4.23 (1H, m, H-6b); Neu: 2.45 (1H, dd, J= 13.9; 5.4 Hz, H-3ec); 1.78 (1H, dd, J= 13.9; 11.5 Hz, H-3ax); 5.57-5.51 (1H, m, H-4); 4.14 (1H, q, J= 10.4 Hz, H-5); 3.68 (1H, dd, J= 10.5; 1.8 Hz, H-6); 5.17-5.10 (1H, m, H-7); 5.19 (1H, ddd, J= 9.1; 25 6.2; 2.8 Hz, H-8); 4.28-4.23 (1H, m, H-9a); 3.92 (1H, dd, J= 12.5; 6.2 Hz, H-9b); 5.97 (1H, d, J= 10.2 Hz, 5-NH); 4.60 (1H, d, J= 15.4 Hz, H-10a); 4.28-4.23 (1H, m, H-10b); **Sph:** 3.61 (1H, dd, J= 10.0; 4.5 Hz, H-1a); 4.02-3.98 (1H, m, H-1b); 4.47 (1H, ddt, J= 11.3; 7.5; 4.1 Hz, H-2); 5.73 (1H, d, J= 9.2 Hz, 2-NH); 5.57-5.51 (1H, m, H-3); 5.48-5.42 (1H, m, H-4); 5.86 (1H, dt, J= 15.2; 6.8 Hz, H-5); 2.00 (2H, m, H-6); 1.38-1.15 30 (22H m, H-7 through H-17); 0.87 (3H 0.87 (3H, t, J= 6.9 Hz, H24).

**MALDI-TOF MS:** [M + Na]⁺ m/z 1807.75 (calculated 1807.93).

### H. Synthesis of O-[3,5-didesoxy-5-hydroxyacetamido-D-glycero-α-D-galacto-2- nonupyranosyl-(2→3)-O-(β-D-galactopyranosyl)-(1→4)-β-D-glucopyranosyl-(1→1)(2R,3S,4E)-2-tetracosanamido-4-octadecene-1,3-diol (formula X) (Figure 1b).

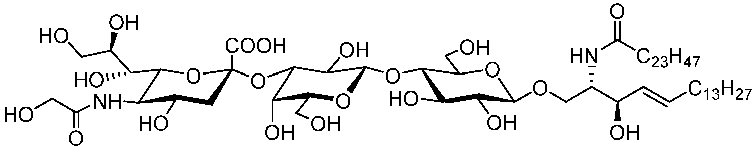

A suspension of **VIII** (1.9 g; 1.06 mmol) in dry MeOH (70 mL) was prepared, a 1.3 M NaOMe/MeOH solution (10.6 mL; 13.8 mmol) was added and stirred for 24 hours at room temperature. H₂ O (18 mL) was added and the mixture was stirred for another 24 hours at room temperature. Upon completion, it was neutralized by stirring with IR-120(H⁺) resin, the resin was removed by filtration, washed with MeOH and the combined filtrates were evaporated to dryness under reduced pressure. The residue was dissolved in H₂ O (200 mL) and the obtained solution was dialyzed at 4 °C for 24 hours against H₂O using a 3.5 KDa membrane. The dialyzed solution was dried by lyophilization at a constant temperature of - 40 °C for 24 hours to obtain ganglioside **X** as a white solid that was homogeneous by CCD (**R_{f}**: 0.56; CHCl₃ /MeOH/ 0.25 % aqueous KCl 25:20:5). Yield: 1.3 g (95 %).

**1H NMR (600 MHz, DMSO-d₆** ): **Glc**: 4.15 (1H, d, J =7.7 Hz, H-1); 3.03 (1H,q, J= 7.6 Hz, H-2); 3.35-3.25 (3H, m, H-3, H-4, H-5); 3.80-3.71 (1H, m, H-6a); 3.64-3.55 (1H, m, H-6b); Gal: 4.18 (1H, d, J= 7.7 Hz, H-1); 3.35-3.25 (2H, m, H-2, H-5); 3.98 (1H, td, J = 9.6; 8.8; 3.7 Hz, H-3); 3.69 (1H, d, J= 3.9 Hz H-4); 3.51-3.42 (2H, m, H-6); **Neu:** 2.752.71 (1H, m, H-3ec); 1.50-1.38 (1H, m, H-3ax); 3.80-3.71 (1H, m, H-4); 3.51-3.41 (2H, m, H-5, H-6); 7.77 (1H, d, J= 7.6 Hz, 5-NH); 3.21 (1H, dd, J= 9.2; 4.4 Hz, H-7); 3.64-3.55 (2H, m, H-8, H-9a); 3.35-3.25 (1H, m, H-9b); 3.92-3.82 (2 H, m, H-10); **Sph:** 3.99 (1H, dd, J= 10.1; 4.5 Hz, H-1a); 3.39 (1H, dd, J= 10.2; 3.6 Hz, H-1b); 3.80-3.71 (1H, m, H-2); 7.49 (1H, d, J= 9.1 Hz, 2-NH); 3.92-3.82 (1H, m, H-3); 5.30 (1H, dd, J= 12.4; 5.6 Hz, H-4); 5.53 (1H, dq, J= 13.0; 6.4 Hz, H-5); 1.93 (2H, hept, J= 7.4 Hz; H-6); 1.34- 1.18 (22H, m, H-7 through H-17); 0.85 (3H, t, J= 6.5 Hz, H-18). FA: 2.01 (2H, q, J= 7.3 Hz, H-2); **1.50-1.38** (2H, m, H-3); 1.34-1.18 (40H, m, H-4 through H-23); 0.85 (3H, t, J= 30 6.5 Hz, H-24).

**MALDI-TOF MS:** [M + Na]⁺ m/z 1304.08 (calculated 1304.66).

### I. Synthesis of O-[3,5-didesoxy-5-hydroxyacetamido-D-glycero-α-D-galacto-2-nonupyranosyl-(2→3)-O-(β-D-galactopyranosyl)-(1→4)-β-D-glucopyranosyl-(1→1)-(2R,3S,4E)-2-tetracos-15-enamido-4-octadecene-1,3-diol (formula XI) (Figure 1b).

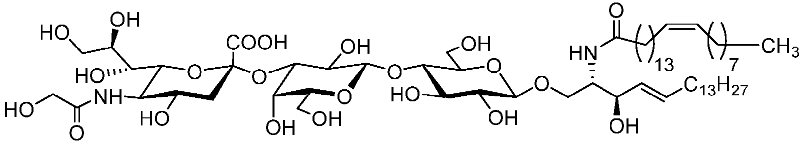

Compound **XI** was obtained by the procedure described above for X from intermediate **IX.** Yield: 1.25 g (95 %).

**1H NMR (600 MHz, DMSO-d₆** ): **Glc**: 4.15 (1H, d, J=7.8 Hz, H-1); 3.07-3.03 (1H, m, H-2); 3.36-3.24 (3H, m, H-3, H-4, H-5); 3.80-3.71 (1H, m, H-6a); 3.65-3.54 (1H, m, H-6b); **Gal:** 4.19 (1H, d, J= 7.7 Hz, H-1); 3.36-3.24 (2H, m, H-2, H-5); 3.96 (1H, dd, J= 9.9; 3.0 Hz, H-3); 3.69 (1H, d, J= 3.6 Hz H-4); 3.52-3.42 (2H, m, H-6); **Neu:** 2.72 (1H,dd, J= 12.1; 4.8 Hz, H-3ec); 1.50-1.37 (1H, m, H-3ax); 3.80-3.71 (1H, m, H-4); 3.52-3.42 (2H, m, H-5, H-6); 7.85 (1H, d, J= 6.9 Hz, 5-NH); 3.23-3.19 (1H, m, H-7); 3.65-3.54 (2H, m, H-8, H-9a); 3.36-3.24 (1H, m, H-9b); 3.92-3.82 (2 H, m, H-10); **Sph:** 4.00 (1H, dd, J = 10.2; 4.6 Hz, H-1a); 3.39 (1H, dd, J= 10.5; 3.2 Hz, H-1b); 3.80-3.71 (1H, m, H-2); 7.49 (1H, d, J = 9.1 Hz, 2-NH); 3.92-3.82 (1H, m, H-3); 5.37-5.31 (1H, m, H-4); 5.53 (1H, dt, J= 14.2; 6.7 Hz, H-5); 1.96-1.88 (2H, m, H-6); 1.34-1.18 (22H, m, H-7 through H-17); 0.85 (3H, t, J= 6.5 Hz, H-18). **FA:** 5.31 (2H, t, J = 5.2 Hz, CH=CH, H-15, H-16); 2.01 (2H, t, J= 7.4 Hz, H-2); 1.97 (2H, q, J= 6.6 Hz, H-14, H-17); 1.50-1.37 (2H, m, H-3); 1.34-1.18 (32H, m, H-4 through H-13, H-18 through H-23); 0.85 (3H, t, J= 6.5 Hz, H24).

**MALDI-TOF MS:** [M + Na]⁺ m/z 1301.94 (calculated 1301.81).

In NMR characterization, the terms Glc, Gal, Neu, Sph and FA refer to the molecular fragments of the compounds glucose, galactose, neuraminyl, sphingosine hydrocarbon chain and hydrocarbon chain provides by the fatty acid, respectively

### Example 2. NGcGM3 d18:1-C24:1 possesses an anti-metastatic effect in liver unlike NGcGM3 extracted from natural source.

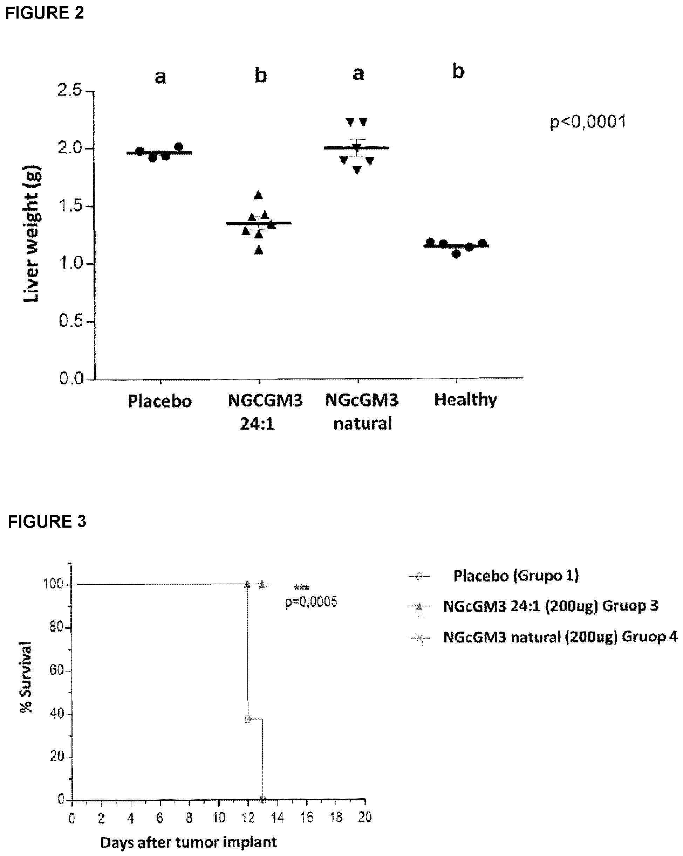

Syngeneic C57BL/6 mice were inoculated intravenously through the tail vein with 2x10⁵ EL-4 thymoma cells. Mice were randomized and distributed into groups of 7-9 animals. Experimental groups received intraperitoneal treatments with 200µg of synthetic and natural variants of NGcGM3 or vehicle (5.6% sucrose solution, 0.75% L-histidine and 0.5% Tween20) on days 1, 5 and 9. Animals were sacrificed 12-13 days after tumor inoculation, livers were removed and weighed to determine the level of hepatic metastatic spread. The liver weight of healthy animals of the same age and batch was used as a control. Figure 2 shows the individual liver weight values of the animals in each group and the mean and standard deviation of the mean. As can be seen, treatment with NGcGM3 d18:1-C24:1 showed a statistically significant antimetastatic effect, so that no differences were observed between the liver weights of tumor-bearing animals treated with NGcGM3 d18:1-C24:1 and healthy animals. Treatment with NGcGM3 extracted from natural source showed no such antimetastatic effect (equal letters p>0.05; different letters p<0.0001; ANOVA and Tukey tests)

### Example 3. Treatment with NGcGM3 d18:1-C24:1 increase survival of EL-4 tumor-bearing animals.

Syngeneic C57BL/6 mice were inoculated intravenously through the tail vein with 2x10⁵ EL-4 thymoma cells (ATCC TIB-39); mice were randomized and distributed into groups of 7-9 animals. Experimental groups received intraperitoneal treatments with 200 µg of ganglioside variants NGcGM3 d18:1-C24:1 and NGcGM3 from natural source or vehicle (5.6% sucrose solution, 0.75% L-histidine and 0.5% Tween20) on days 1, 5 and 9. The animals were monitored and sacrificed if they presented any sign or symptom that affected their ability to ingest food and water or compromised their general state of health, according to veterinary criteria. Figure 3 shows on day 13 after tumor cells were inoculated, all mice in the groups treated with vehicle solution (Placebo) or with NGcGM3 from natural source had died, while all the animals treated with NGcGM3 d18:1-C24:1 were alive and healthy. Hence, it is observed that treatment with NGcGM3 d18:1-C24:1 significantly increased the survival of EL-4 tumor-bearing animals (p=0.0005, Long-rank Mantel-Cox, chi-square tests).

### Example 4. P3X63 murine myeloma cells express CD1d in the surface.

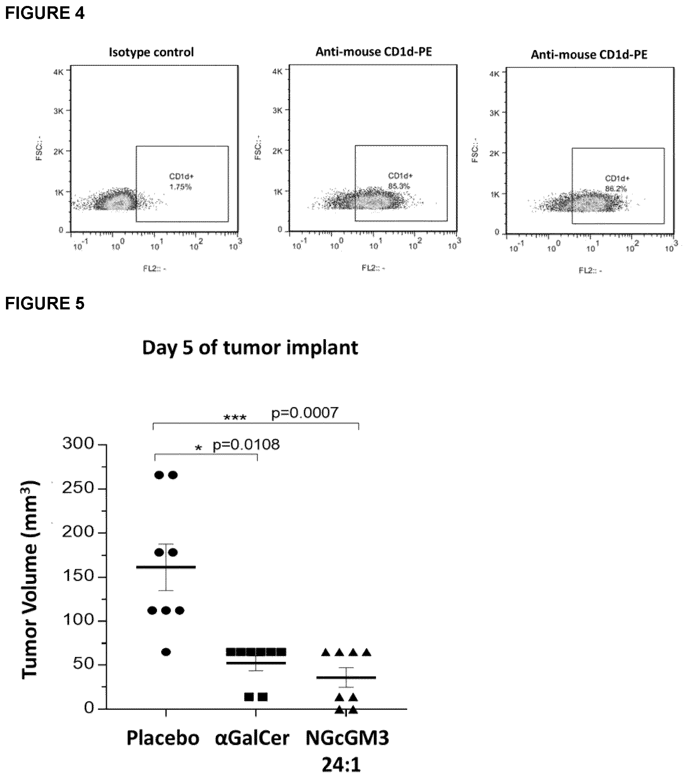

CD1d expression in the murine myeloma cell line, P3X63 (P3X63Ag8.653, CRL 1580) was assessed by flow cytometry using a phycoerythrin-conjugated mouse anti-CD1d monoclonal antibody (clone 1B1, eBioscience). Cells were pre-incubated with α-CD16/32 monoclonal antibody to block nonspecific binding, and a fluorophore-conjugated antibody isotype (ARL2397; BioSource) was used as a control. Cells were acquired on a Sysmex Partec flow cytometer (Sachsen, Germany) and analysis was performed in FlowJo 10 software (Tree Star, USA). As can be seen in Figure 4, about 90% of P3X63 cells express CD1d on their surface.

### Example 5. Administration of NGcGM3 d18:1-C24:1 inhibits tumor growth of high P3X63 myeloma loads, even better than αGalCer.

1x10⁶ P3X63 cells were implanted subcutaneously into syngeneic BALB/c mice. Animals were randomized, divided into groups of 6-8 and received intraperitoneal treatments with 200 µg NGcGM3 d18:1-C24:1 ganglioside, αGalCer or vehicle (5.6% sucrose solution, 0.75% L-histidine and 0.5% Tween20 0.5%) on days 1, 5 and 9. Tumor diameters were measured with a caliper and tumor volume (TV) was calculated according to the formula: TV (mm³) = π/6 × major diameter × (minor diameter)². Figure 5 shows the mean TV and the distribution of individual measurements per group at day 5 of tumor cell implantation. The individual tumor volume values of the animals in each group and the mean and standard deviation of the mean in each case on day 5 of the experiment are shown. Table 1 shows the percentage distribution of the tumor volume of the animals in each group.

**Table 1. Percentage distribution of tumor volume of the animals in each group.**

| | **Placebo** | **αGalCer** | **NGcGM3 24:1** |
|---|---|---|---|
| **TV > 36 mm³** | 100% | 75% | 50% |
| **TV < 36 mm³** | - | 25% | 50% |

Treatments with NGcGM3 d18:1-C24:1 and αGalCer significantly inhibited the growth of subcutaneous tumors, although with different levels of statistical significance (p<0.001 for NGcGM3 d18:1-C24:1 and p<0.05 for αGalCer, ANOVA and Tukey tests). In addition, in the group treated with NGcGM3 d18:1-C24:1, 50 % of the animals presented tumors with a TV less than 36 **mm³** compared to the group treated with αGalCer in which 25 % of tumors had less than 36mm³. In the placebo group tumors with TV greater than 36 mm³ were present in all animals.

### Example 6. NGcGM3 ganglioside d18:1-C24:1 shows a potent stimulatory effect on iNKT cells in vitro, superior to other synthetic variants of NGcGM3 and to the ganglioside purified from erythrocytes.

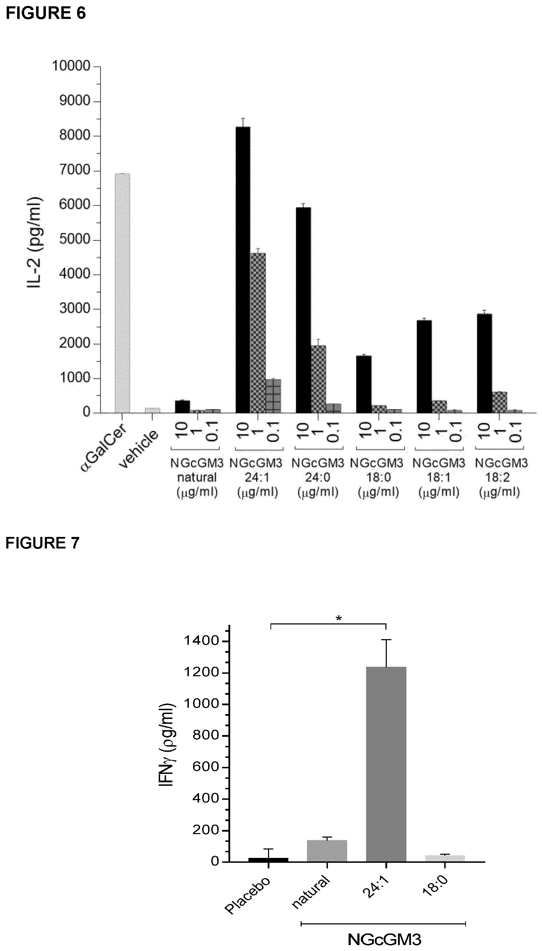

5x10⁴ bmDC were grown in RPMI-1640 medium with 10% FBS in the presence of αGalCer at a concentration of 100 ng/ml and concentrations of 0.1, 1 and 10 µg/ml of natural NGcGM3 and the synthetic variants NGcGM3 d18:1-C24:1, NGcGM3 d18:1-C24:0, NGcGM3 d18:1-C18:0, NGcGM3 d18:1-C18:1 and NGcGM3 d18:1-C18:2 or vehicle (0.1 % DMSO) for 16 -24 hours. Then, 10⁵ FF13 hybridoma cells were added and co-cultured with the bmDCs for 24 hours. The culture supernatants were collected and evaluated for IL-2 content by ELISA. Figure 6 shows that the synthetic variants of NGcGM3 at the highest concentration tested (10 µg/ml) were able to activate the iNKT cell hybridoma in contrast to the NGcGM3 ganglioside extracted from natural source, which at none of the tested concentrations stimulated IL-2 secretion by iNKT cells. Differences were observed between the NGcGM3 variants in terms of their antigenicity to iNKT cells based on the length and structure of the fatty acid in the ceramide. The d18:1-C24:1NGcGM3 variant showed a potent activation of FF13 hybridoma cells, being the only variant able to activate iNKT cells at the same concentration of αGalCer (100 ng/ml) and to surpass the effect of αGalCer at the concentration of 10 µg/ml (p< 0.05, ANOVA and Tukey tests).

### Example 7. Administration of NGcGM3 d18:1-C24:1 activates iNKT cells in vivo while NGcGM3 d18:1-C 18:0 and NGcGM3 extracted from erythrocytes are inactive.

C57BL/6 mice were injected intraperitoneally with 200 µg of natural NGcGM3 ganglioside, NGcGM3 d18:1-C24:1, NGcGM3 d18:1-C 18:0 and 2 µg of αGalCer or vehicle (5.6 % sucrose, 0.75 % L-histidine and 0.5 % Tween-20) and blood was drawn 16 hours after the administration. Serum IFNγ levels were assessed by ELISA as an indirect measure of iNKT cell activation (Mouse IFNy ELISA Ready-SET-Go!, eBioscience). Figure 7 shows that in mice receiving NGcGM3 d18:1-C24:1, IFNy levels were higher than in the rest of the experimental groups and statistically higher (p < 0.05) than in animals in the placebo group (Kruskal-Wallis, Dunn). This result suggests an *in vivo* activation of iNKT cells mediated by NGcGM3 d18:1-C24:1.

### Example 8. Synthetic variants of ganglioside NGcGM3 have a greater stimulatory effect on iNKT cells than synthetic variants of ganglioside NAcGM3 with identical ceramide structure.

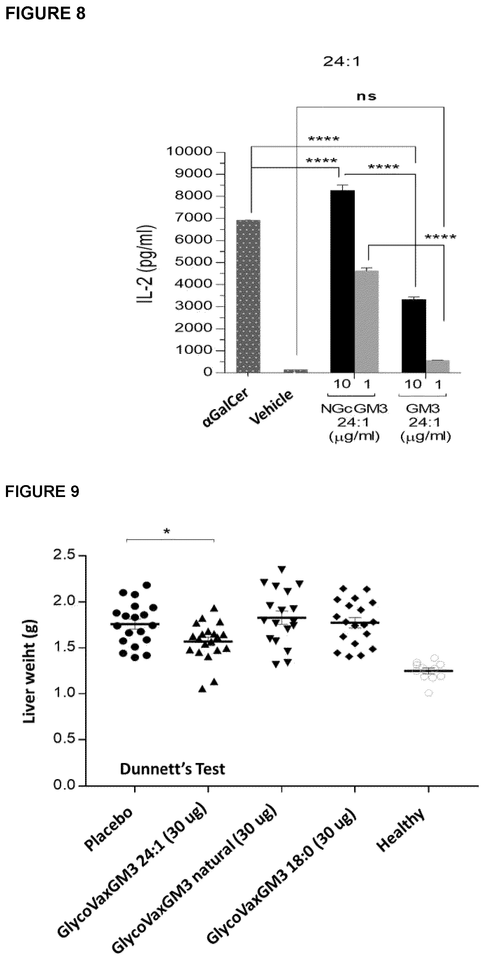

5x10⁴ bmDC were cultured in RPMI-1640 medium with 10% FBS in the presence of 10 µg/ml and 1 µg/ml of NGcGM3 d18:1-C24:1 ganglioside, GM3 d18:1-C24:1 or vehicle (0.1% DMSO) for 16-24 hours. Next, 10⁵ FF13 hybridoma cells were added and co-cultured with the bmDCs for 24 hours. The culture supernatants were collected and IL-2 content quantified by ELISA. From Figure 8, the synthetic ganglioside variants NGcGM3 and GM3, with identical ceramide structure, have differences in their ability to stimulate iNKT cells. The NGcGM3 variant shows a more potent effect on iNKT cell activation than the GM3 variant (ANOVA, Tukey tests).

### Example 9. Nanoparticles of NGcGM3 d18:1-C24:1 and OPMC of N. meningitidis exhibit a potent anti-metastatic effect in liver in contrast to nanoparticles formed with NGcGM3 d18:1-C18:0 or NGcGM3 extracted from erythrocytes.

Syngeneic C57BL/6 mice were inoculated intravenously through the tail vein with 2x10⁵ EL-4 thymoma cells; mice were randomized and distributed into groups of 7-9 animals. Experimental groups received intraperitoneal treatments with 10-30 µg of nanoparticles of NGcGM3 d18:1-C24:1, NGcGM3 d18:1-C18:0 and natural NGcGM3 with OMPC of *N. meningitidis* on days 1, 5 and 9 or Tris/HCl solution (Placebo). Animals were sacrificed 12-13 days after tumor inoculation. To determine the level of liver metastatic spread, their livers were removed and weighed. Figure 9 shows the individual liver weight values of the animals in each group and the mean and standard deviation of the mean. Noteworthy, treatment with nanoparticles containing NGcGM3 d18:1-C24:1 ganglioside showed a statistically significant antimetastatic effect with respect to the placebo group, while the groups treated with nanoparticles containing NGcGM3 d18:1-C18:0 ganglioside or the ganglioside obtained from natural source did not show such effect (equal letters p>0.05; different letters p=0.0015; ANOVA and Tukey tests).

### Example 10. Administration of NGcGM3 d18:1-C24:1 incorporated into nanoparticles with N. meningitidis OMPC inhibits the growth of high tumor burdens of P3X63 murine myeloma even better than αGalCer and systemic NGcGM3 d18:1-C24:1.

1x10⁶ P3X63 myeloma cells were implanted subcutaneously into syngeneic BALB/c mice. Animals were randomized, divided into groups of 6-8 and received treatments with vehicle solution (5.6% sucrose, 0.75% L-histidine and 0.5% Tween-20), αGalCer, NGcGM3 d18:1-C24:1 or nanoparticles of OMPC and NGcGM3 d18:1-C24:1 (GlycoVax 24:1) on days 1, 5 and 9.

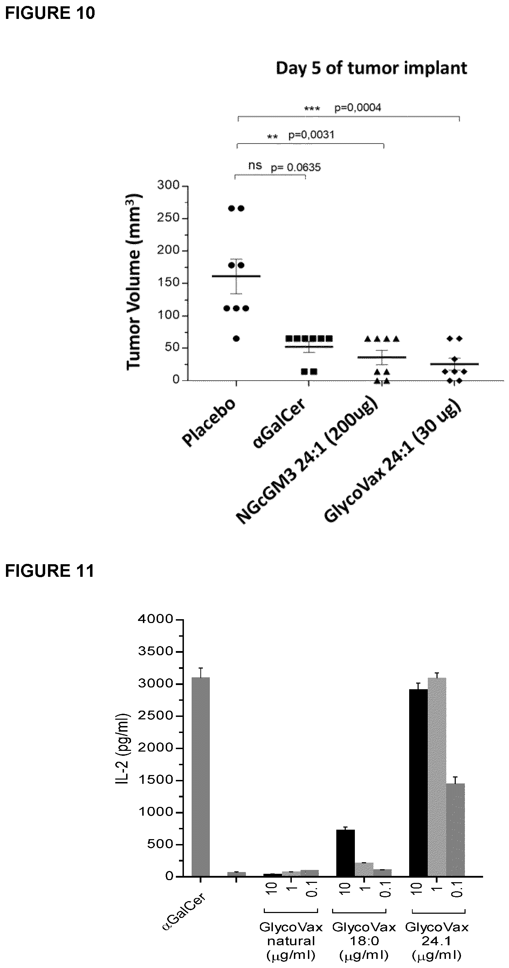

Subcutaneous tumor diameters were measured every 2-3 days with a caliper and TV was calculated according to the formula: TV (mm³) = π/6 × major diameter × (minor diameter)². Figure 10 shows the TV of individual animals in each group and the mean and standard deviation of the mean in each case at day 5 of tumor cell implantation, at which time all animals in the placebo group had measurable tumors. In contrast to treatment with αGalCer that did not show a statistically significant antitumor effect, treatments with NGcGM3 d18:1-C24:1 and GlycoVax 24:1 showed significant inhibition of tumor growth with respect to the control group (p=0.0031 and p=0.0004 respectively, nonparametric ANOVA, Kruskal-Wallis, Dunn's multiple comparison test).

Table 2 shows the percentage distribution of the tumor volume of the animals in each group.

**Table 2. Percentage distribution of tumor volume of animals in each group.**

| | **Placebo** | **αGalCer** | **NGcGM3 24:1** | **GlycoVax 24:1** |
|---|---|---|---|---|
| **TV > 36 mm³** | 100% | 75% | 50% | 25% |
| **TV < 36 mm³** | - | 25% | 50% | 75% |

Treatment with GlycoVax 24:1 showed the strongest antitumor effect with 75 % of animals with tumors below 36 mm³. For the group treated with systemic NGcGM3 d18:1-C24:1 ganglioside , 50 % of animals with tumors below 36 mm³ were observed. Treatment with αGalCer reduced tumor volume below 36 mm³ in 25 % of animals.

### Example 11. Nanoparticles of NGcGM3 d18:1-C24:1 and OMPC of N. meningitidis activate iNKT cells unlike nanoparticles obtained from incorporation of NGcGM3 d18:1-C18:0 or NGcGM3 extracted from natural source.

5x10⁴ bmDC were cultured in RPMI-1640 medium with 10% FBS in the presence of 100 ng/ml, 1 µg/ml or 10 µg/ml concentrations of nanoparticles, αGalCer or vehicle (0.1% DMSO) for 16 -24 hours. The bmDCs were then co-cultured with 10⁵ FF13 hybridoma cells for 24 hours. The culture supernatants were collected and IL-2 content quantified by ELISA. From

Figure 11, nanoparticles containing NGcGM3 d18:1-C24:1 ganglioside (GlycoVax 24:1) activated the iNKT cell hybridoma similarly than αGalCer at the concentrations of 10 µg/ml and 1 µg/ml, and such effect was maintained at the concentration of 100 ng/ml. In the case of nanoparticles obtained with NGcGM3 ganglioside extracted from a natural source, no activation of the iNKT cell hybridoma was observed at any of the tested concentrations and with nanoparticles containing NGcGM3 d18:1-C18:0 ganglioside only a moderate effect was observed at the highest evaluated concentration (10 µg/ml).

### Example 12. Adoptive transfer of dendritic cells incubated with NGcGM3 d18:1C24:1 generates a potent anti-metastatic effect in liver while transfer of dendritic cells incubated with NGcGM3 extracted from erythrocytes are ineffective.

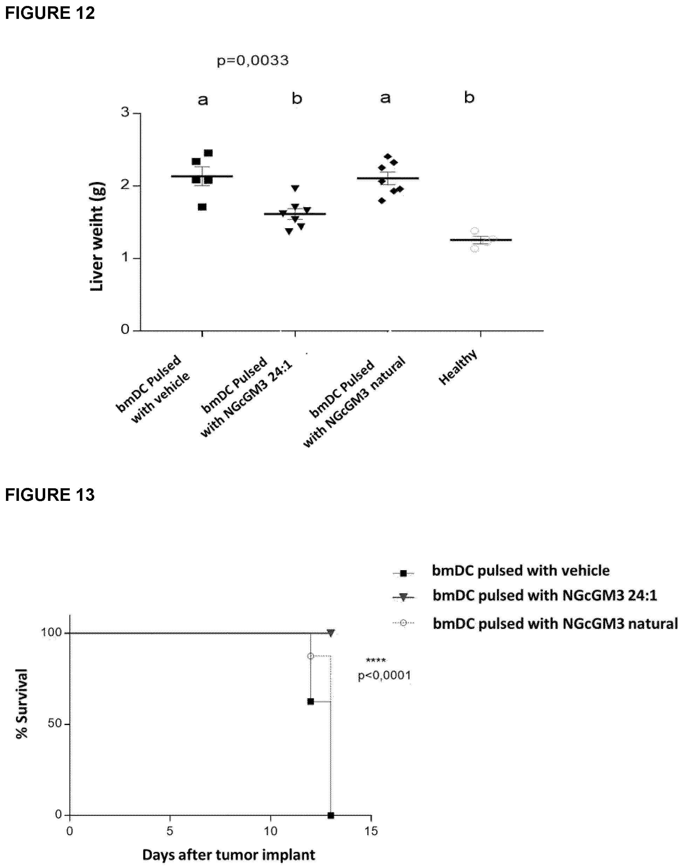

Syngeneic C57BL/6 mice were inoculated intravenously through the tail vein with 2x10⁵ EL-4 thymoma cells; mice were randomized and distributed into groups of 7-9 animals. On the day following tumor inoculation, 6x10⁵ bmDC, previously cultured for 16 hours with vehicle (DMSO 0.1%), NGcGM3 d18:1-C24:1 ganglioside (10 µg/ml) or NGcGM3 extracted from natural source (10 µg/ml) were administered in a single dose, intravenously. Cells were extensively washed with serum-free RPMI-1640 medium to remove excess of lipids before transfer. Animals were sacrificed 12-13 days after tumor inoculation, livers were collected and the level of hepatic metastatic spread was determined by weigh. Livers from healthy mice of the same age and batch were used as a reference. Figure 12 shows the individual liver weight values of the animals in each group and the mean and standard deviation of the mean. Clearly, the transfer of bmDC pulsed with NGcGM3 d18:1-C24:1, induce a significant reduction of EL-4 liver metastasis, compared to the placebo group, in which empty dendritic cells were transferred. Similarly, the transfer of bmDC incubated with ganglioside extracted from a natural source was ineffective. (equal letters p>0.05, different letters p<0.05, ANOVA and Tukey tests).

### Example 13. Adoptive transfer of dendritic cells incubated with NGcGM3 d18:1C24:1 increase survival of tumor-bearing animals in contrast to transfer of dendritic cells incubated with NGcGM3 extracted from erythrocytes.

Syngeneic C57BL/6 mice were inoculated intravenously through the tail vein with 2x10⁵ EL-4 thymoma cells; mice were randomized and distributed into groups of 7-9 animals. On the day following tumor inoculation, 6x10⁵ bmDC previously cultured for 16 hours with vehicle (DMSO 0.1%), NGcGM3 d18:1-C24:1 ganglioside (10 µg/ml) or the ganglioside extracted from a natural source (10 µg/ml) were administered in a single dose, intravenously. Cells were washed extensively with serum-free RPMI-1640 medium to remove excess of lipids before transfer. Mice were monitored for signs or symptoms that affected their ability to ingest food and water or compromised their general health status, according to veterinary criteria. From Figure 13, 13 days after tumor cells inoculation, all animals in the placebo group, receiving empty dendritic cells and all animals that received cells pulsed with NGcGM3 of natural origin had died. In contrast, in the group receiving bmDC exposed to NGcGM3 d18:1-C24:1, all animals (100%) were alive and healthy. Statistically different survival curves indicate a significant increase (p<0.0001) in survival for animals treated with bmDC previously cultured in the presence of NGcGM3 d18:1-C24:1 (Long-rank Mantel-Cox, chi-squared tests).

### Example 14. Adoptive transfer of dendritic cells incubated with NGcGM3 d18:1-C24:1 reduces tumor growth in P3X63 murine myeloma tumor-bearing animals in contrast to transfer of dendritic cells incubated with NGcGM3 extracted from erythrocytes.

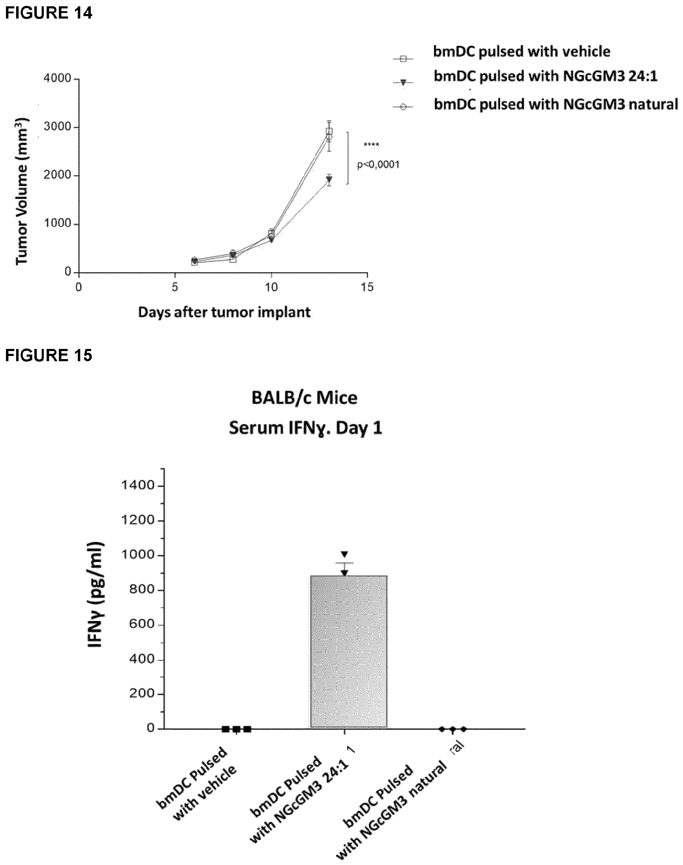

1x10⁶ P3X63 cells were implanted subcutaneously into syngeneic BALB/c mice. Animals were randomized, divided into groups of 6-8 and on the day following implantation received a single intravenous dose of 6x10⁵ bmDC previously cultured for 16 hours with vehicle (DMSO 0.1%), NGcGM3 d18:1-C24:1 ganglioside (10 µg/ml) or the ganglioside extracted from natural source (10 µg/ml). Cells were washed extensively with serum-free RPMI-1640 medium to remove excess of lipids before transfer. Tumor growth was followed by measuring tumor diameters with a caliper and TV was determined according to the formula: TV (mm³ ) = π/6 × major diameter × (minor diameter)² . Figure 14 shows that the transfer of bmDC pulsed with NGcGM3 d18:1C24:1 induce an antitumor effect by significantly (p<0.0001) reducing tumor growth with respect to the group treated with empty bmDC. When bmDC incubated with the ganglioside extracted from a natural source were transferred, no antitumor effect was observed (ANOVA and Tukey tests).

### Example 15. Activation of NKT cells in vivo in BALB/c mice occurs with transfer of dendritic cells incubated with NGcGM3 d18:1-C24:1 as opposed to dendritic cells incubated with NGcGM3 extracted from erythrocytes.

1x10⁶ P3X63 cells were implanted subcutaneously into syngeneic BALB/c mice. Animals were randomized, divided into groups of 6-8 and on the day following implantation received a single intravenous dose of 6x10⁵ bmDC previously cultured for 16 hours with vehicle (DMSO 0.1%), NGcGM3 d18:1-C24:1 (10 µg/ml) or the ganglioside extracted from a natural source (10 µg/ml). Cells were extensively washed with serum-free RPMI-1640 medium to remove excess of lipids before transfer. Sixteen hours after transfer, blood was drawn from 2-3 animals from each group and serum IFNγ levels were assessed by ELISA. Figure 15 shows that both empty bmDC transfer and bmDC transfer pulsed with NGcGM3 extracted from erythrocytes failed to stimulate early IFNy secretion. However, in animals transferred with bmDC previously cultured in the presence of NGcGM3 d18:1-C24:1, significant levels of IFNy were detected at 16 hours of treatment. This early secretion of IFNγ is associated with the activation of iNKT cells.

### Example 16. Activation of NKT cells in vivo in C57BL/6 mice occurs with transfer of dendritic cells incubated with NGcGM3 d18:1-C24:1 as opposed to dendritic cells incubated with NGcGM3 extracted from erythrocytes.

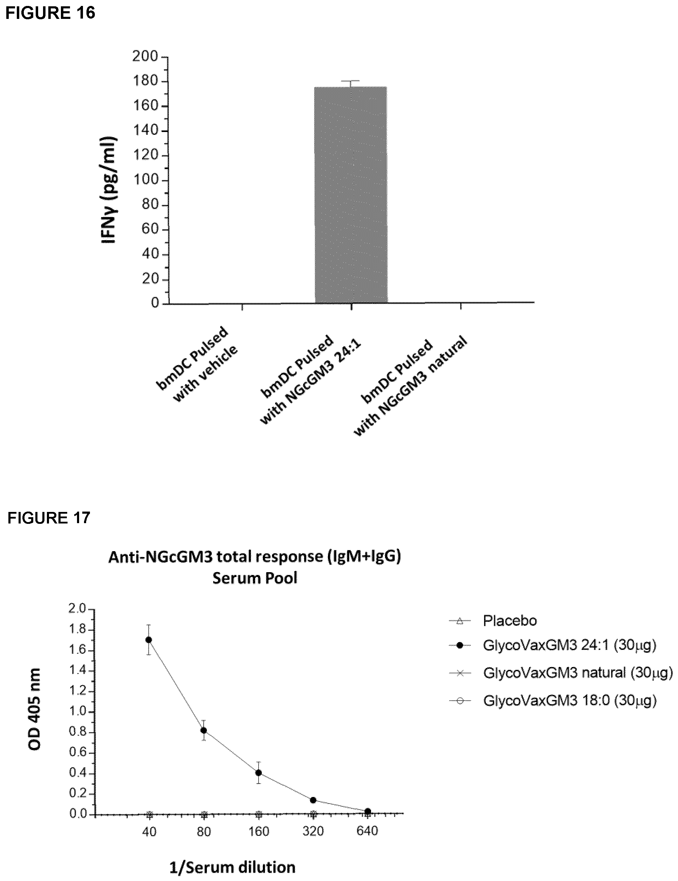

Syngeneic C57BL/6 mice were inoculated intravenously through the tail vein with 2x10⁵ EL-4 thymoma cells; mice were randomized and distributed into groups of 7-9 animals. On the day following tumor inoculation, 6x10⁵ bmDC previously cultured, for 16 hours with vehicle (DMSO 0.1%), NGcGM3 d18:1-C24:1 (10 µg/ml) or the ganglioside extracted from a natural source (10 µg/ml) were administered in a single dose, intravenously. Cells were washed extensively with serum-free RPMI-1640 medium to remove excess of lipids before transfer. Sixteen hours after transfer, blood was drawn from 2-3 animals from each group and serum IFNγ levels were assessed by ELISA. In Figure 16, transfer of bmDC pulsed with vehicle and transfer of bmDC pulsed with a natural source NGcGM3 did not activate iNKT cells, whereas transfer of bmDC previously pulsed with NGcGM3 d18:1-C24:1 induced the activation of iNKT cells in terms of early IFNy secretion.

### Example 17. NGcGM3 d18:1-C24:1 incorporated into nanoparticles with N. meningitidis OMPC, when administered to mice, induces a strong specific antibody response, unlike when NGcGM3 d18:1-C18:0 and NGcGM3 extracted from erythrocytes are incorporated.

Syngeneic C57BL/6 mice were inoculated intravenously through the tail vein with 2x10⁵ EL-4 thymoma cells, randomized and distributed into groups of 7-9 animals. Experimental groups received intraperitoneal treatments with 10-30 µg of nanoparticles with *N. meningitidis* OMPC, containing NGcGM3 d18:1-C24:1, NGcGM3 d18:1-C18:0 or NGcGM3 extracted from a natural source on days 1, 5 and 9 or Tris/HCl solution (Placebo). On day 13, blood was drawn from 3-4 animals per group and the specific IgM and IgG antibody response against NGcGM3 was assessed by ELISA. For this purpose, a single pool of sera per group was prepared from the sera of the individual animals in each case. For NGcGM3-specific antibody detection and titration by ELISA, NUNC PolySorp 96-well plates were coated with 0.16 nmol/well of NGcGM3 (of natural origin) dissolved in methanol. The solvent was evaporated 1-2 hours at 37° C and a 16-24 hours blocking step was performed with 1% (m/v) lipid-free bovine serum albumin fraction V in phosphate buffered saline (PBS). Subsequently, sera were added in the dilution range evaluated and after washes with PBS-Tween20 0.05% (v/v) biotinylated goat anti-IgM + mouse IgG antibodies (Sigma) was added at 1/5000 dilution in blocking solution. After incubation for 1 hour at 37° C alkaline phosphatase-conjugated streptavidin (Jackson Immnoresearch) was added at a 1/2000 dilution in blocking solution. Following 1 hour at 37° C the enzymatic reaction was visualized with p-nitrophenyl phosphate (PNPP) at 1 mg/ml, dissolved in 1 M diethanolamine buffer, pH 9.6 and MgCl₂ 1 mM. Absorbance was read at 405 nm. To eliminate the effect of non-specific signals, the sera were also analyzed in empty wells containing only methanol. Absorbance at each serum dilution were corrected by substracting the values from empty wells. The serum titer was defined as the inverse of the highest dilution that yielded a final absorbance value greater than 0.1. Figure 17 shows that an anti-NGcGM3 antibody response was only present in the animals injected with the nanoparticles containing NGcGM3 d18:1-C24:1. The obtained IgM and IgG antibody titer of 1/320, specific for NGcGM3 could be considered outstanding due to the lipidic nature this antigen and the short time frame of only 13 days. Nanoparticles formulated with the ganglioside extracted from a natural source or NGcGM3 d18:1-C18:0, did not generate anti-NGcGM3 antibody responses.

### Example 18. The antitumor activity of NGcGM3 d18:1-C24:1 ganglioside is NKT cell-dependent.

Syngeneic C57BL/6 mice were treated intraperitoneally with 1 mg/200µL PBS of anti-NK1.1 depleting antibody (clone PK136, ATCC) or 20 µL/200µL PBS of polyclonal anti-asialoGM1 antibody (OriGene Technologies). Treatment with the anti-NK1.1 antibody eliminate NK and NKT cell populations by more than 90 %, while the anti-asialoGM1 antibody is used to selectively deplete the NK cell population. Forty-eight hours after antibody treatment, 2x10⁵ EL-4 thymoma cells were inoculated intravenously into all animals, including two groups of mice that had been administered 200 µl of PBS intraperitoneally as a control. On days 1, 5 and 9 after tumor inoculation, the experimental groups received intraperitoneal treatment with 200 µg of NGcGM3 d18:1-C24:1 or vehicle (Placebo Group) according to the following distribution of experimental groups:
Group 1: Placebo + PBS
Group 2: NGcGM3 d18:1-C 24:1, 200 µg/200 µL + PBS
Group 3: Placebo + αNK1.1
Group 4: NGcGM3 d18:1-C24:1, 200 µg/200 µL + αNK1.1
Group 5: Placebo + α-asialoGM1
Group 6: NGcGM3 d18:1-C24:1, 200 µg/200 µL + α-sialoGM1

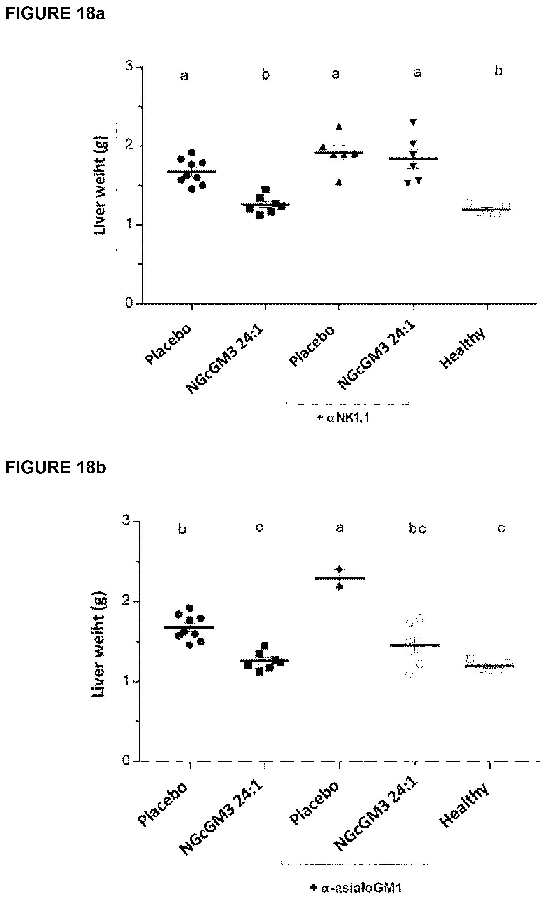

To maintain the depletion of the populations of interest throughout the duration of the experiment, equal doses of depleting antibodies were administered on days 4 and 11 after tumor inoculation. Animals were sacrificed 12-13 days after tumor inoculation, livers were removed and the level of hepatic metastatic spread was determined by weigh. Livers from healthy mice of the same age and batch were used as a reference. Figure 18a shows the effect of depletion of NKT and NK cell populations on the antitumor effect of NGcGM3 d18:1-C24:1 treatment. While the anti-metastatic effect of NGcGM3 d18:1-C24:1 treatment is present in mice in which the NK and NKT cell populations were not affected, the absence of these populations in animals treated with the anti-NK1.1 Mab abolishes the aforementioned anti-tumor effect (equal letters p>0.05; different letters p<0.0001; ANOVA and Tukey tests). This result suggests that the antitumor effect of NGcGM3 d18:1-C24:1 administration is dependent on NK or NKT cells. However, when evaluating the antitumor effect of NGcGM3 d18:1-C24:1 administration in animals that had only the NK cell population removed (Figure 18b), it is observed that despite population depletion, the antitumor effect is maintained (equal letters p>0.05; different letters p<0.0001; ANOVA and Tukey tests), indicating that this population is not directly responsible for the observed antimetastatic effect. Taken together, these results suggest that the anti-tumor effect of NGcGM3 d18:1-C24:1 is primarily dependent on NKT cell activity.

## Claims

1. A synthetic ganglioside of NGcGM3 represented by the formula: 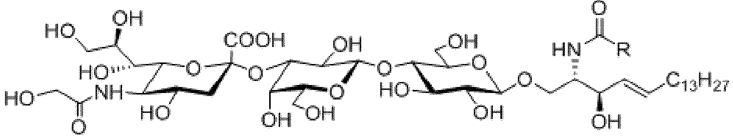 and corresponding to the anomeric α-trisaccharide, wherein R is selected from the group comprising:
- C23H47 and
- C23H45.

2. A pharmaceutical composition comprising as active substance the ganglioside according to claim 1 and a pharmaceutically acceptable vehicle.

3. The composition according to claim 2 containing another immunomodulator.

4. The composition of any one of claims 2-3 containing an antigen.

5. The composition according to claim 2 in the form of nanoparticles or liposomes.

6. The composition according to claim 5 wherein the nanoparticles are formed by hydrophobic insertion of one or more of the gangliosides of any one of claims 1-2 into hydrophobic outer membrane proteins of gram-negative bacteria.

7. The composition of claim 6 wherein the hydrophobic outer membrane proteins are from the bacterium Neisseria meningitidis.

8. Use of the ganglioside of claim 1 in the manufacture of a medicament for the treatment of cancer and its metastases.

9. Use of the pharmaceutical compositions of any one of claims 2-7 in the manufacture of a medicament for the treatment of cancer and its metastases.

10. A method for treating a mammal suffering cancer comprising administering to the mammal a therapeutically effective amount of the ganglioside of claim 1 with activity against that malignant tumor and its metastases.

11. The method according to claim 10 wherein the tumor is CD1d positive or CD1d negative.

12. The method according to claim 10 wherein the metastases are hepatic.

13. The method of any one of claims 10-12 wherein the compound is provided as a nanoparticle or liposome.

14. The method of any one of claims 10-12 wherein the mammal is a human.

15. An in vitro method for preparing dendritic cells loaded with the ganglioside of claim 1, comprising:
a) incubate dendritic cells obtained from a mammal with said gangliosides,
b) to use the cells obtained in adoptive cell transfer therapies.

16. An in vitro method for the preparation of dendritic cells loaded with the pharmaceutical compositions of any one of claims 2-6 comprising:
a) incubate dendritic cells obtained from a mammal with said compositions,
b) to use the cells obtained in a) in adoptive cell transfer therapies.

17. The method of any one of claims 15-16 wherein the mammal is a human.
